# EUROPEAN PATENT SPECIFICATION

(11) **EP 1 874 261 B1**
(45) Date of publication and mention of the grant of the patent: **24.11.2010**
(21) Application number: 06751123.8
(22) Date of filing: 21.04.2006
(51) Int. Cl.: A61K 8/02, A61Q 17/04, G01N 21/64

(54) **TOPICAL COMPOSITION DETECTION**
NACHWEIS EINER TOPISCHEN ZUSAMMENSETZUNG
DETECTION DE COMPOSITION TOPIQUE

(30) Priority: 29.04.2005 US 119260
(43) Date of publication of application: 09.01.2008
(60) Divisional application: 10177464.4
(73) Proprietor: JOHNSON & JOHNSON CONSUMER COMPANIES, INC., Skillman, New Jersey 08558 (US)
(72) Inventor: COLE, Curtis, Ringoes, New Jersey 08551 (US); NIKIFOROS, Kollias, Skillman, New Jersey 08558 (US)
(74) Representative: Kirsch, Susan Edith
(86) International application number: PCT/US2006/015314
(87) International publication number: WO 2006/118835

(56) References cited:
- EP-A- 0 913 681
- EP-A- 1 099 437
- EP-A2- 1 277 461
- WO-A-00/46233
- WO-A-02/00190
- WO-A-2005/009604
- WO-A-2005/011622
- WO-A-2005/067883
- DE-A1-102004 002 601
- DE-A1-102004 002 601
- DE-A1-102004 002 606
- DE-A1-102004 002 606
- US-A- 2 949 403
- US-A- 4 434 154
- US-A- 5 543 137
- US-A- 5 626 839
- US-B1- 6 313 181
- STOKES R P ET AL: "The feasibility of using fluorescence spectroscopy as a rapid, non-invasive method for evaluating sunscreen performance" JOURNAL OF PHOTOCHEMISTRY AND PHOTOBIOLOGY B BIOLOGY, vol. 50, no. 2-3, June 1999 (1999-06), pages 137-143, XP002395308 ISSN: 1011-1344 cited in the application

## Description

The present invention relates to compositions comprising topical agents and fluorescent chromophores, as well as methods and devices for determining the presence of such compositions on a surface, such as skin.

### Background of the Invention

Cosmetics such as skin and hair care compositions, sunscreens and the like provide a variety of benefits. However, the benefits of such products depend in large measure on use of correct amount. For example, sunscreens provide significant protection against both acute and chronic damage to the skin from solar UV radiation. In order to receive such protection, the consumer must apply the correct amount of sunscreen. Studies have shown that consumers chronically underapply sunscreen, and thus limit the benefit of its use.

Accordingly, there exists a need for a simple, user-friendly system that would enable a consumer to determine whether he is wearing an appropriate amount of product, i.e. a topical composition such as a sunscreen. One approach to such a system, as discussed by Stokes, et al., "The Feasibility of Using Fluorescence Spectroscopy As a Rapid Invasive Method For Evaluating Sunscreen Performance", J. Photochemistry and Photobiology Biology, 50:137-143 (1999) employs a topical composition that includes a UV-sunscreen "active ingredient" that undergoes autofluorescence. However, in such as system where the source of fluorescence is from an active ingredient responsible for absorbing ultraviolet radiation, overall system performance is poor. In practice, too large a percentage of the fluorescence emission is absorbed by the active ingredient, resulting in low fluorescence signal.

Another approach (also discussed by Stokes, et al.) involves the use of a composition that includes a UV- absorbing compound and a separate fluorescent chromophore; and a device to detect the presence of the fluorescent chromophore. However, as the authors note, "none of the substances used in the present study is ideal for this purpose; some do not mix readily with sunscreen products, while the fluorescence of others is quenched by the active ingredients present in sunscreens." Because of these drawbacks, it is not practical, using systems of the prior art, to accurately determine the level of an "active ingredient" (such as a sunscreen) on the skin once the composition is applied. Similarly, it is not possible, using systems of the prior art, to determine accurately whether or to what degree the UV filter has been rendered ineffective or removed, such as by the gradual wearing away by washing with water or abrading away.

It would also be desirable to use a fluorescent chromophore at a concentration in a topical composition that will not create an objectionable color on the skin. However, at the same time, the fluorescent chromophore must be present in a concentration that is detectable.

In addition, it would also be desirable to have a system capable of accurately determining whether more than one topical composition, and, in particular, compositions with various functions (e.g., recreational sunblock, moisturizers with sun protection) are present in sufficient amount on the skin.

Furthermore, it is desirable that any fluorescent marker used be safe enough to be topically applied to the skin without deleterious biological effects.

It has now been discovered that introduction of particular fluorescent chromophores into a topical composition may be coupled with a device for detection of the fluorescent chromophore. In one embodiment, the fluorescent chromophore has an absorbance at the wavelength of its excitation that is considerably greater the absorbance of the topical agent at that same wavelength. In another embodiment, the fluorescent chromophore is coupled with an ultraviolet sunscreen agent and has a water solubility less than about 1% by weight and a wavelength of emission greater than about 400 nm. Applicants have also developed a device for determining the presence of a composition comprising a topical agent and a fluorescent chromophore on a surface such as the skin, as well as kits comprising the same.

The result is a system that allows a consumer to determine the presence and amount of the composition on a surface, such as his hair, skin, nails or genital areas.

### Summary of the Invention

The invention provides a composition comprising one or more oil soluble ultraviolet sunscreen agents and a fluorescent chromophore, wherein the fluorescent chromophore is oil soluble and has a wavelength of excitation greater than 400nm.

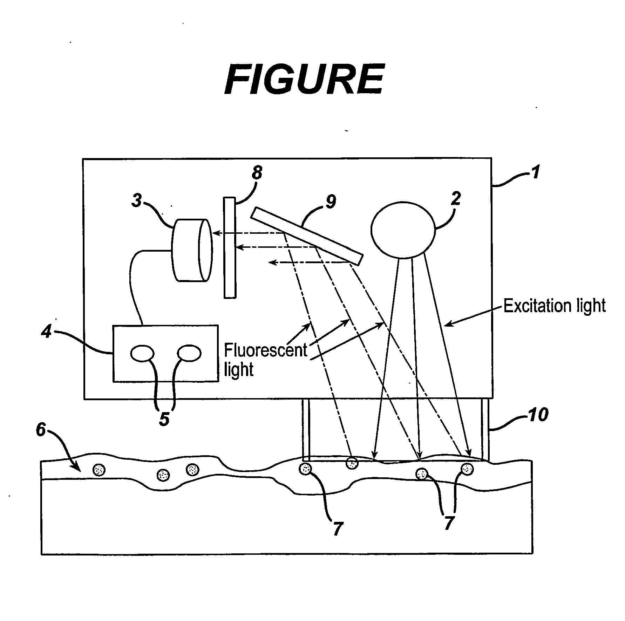

### Brief Description of the Drawing

The Figure depicts a device according to the invention.

### Detailed Description of the Invention

Unless defined otherwise, all technical and scientific terms used herein have the meaning commonly understood by one of ordinary skill in the art to which the invention pertains. As used herein, compounds include all isomers thereof (e.g., tocopherol) unless otherwise indicated.

The composition of the invention comprises one or more topical agents. As used herein, a topical agent is a compound that offers a cosmetic, pharmaceutical, or therapeutic benefit when topically administered to the hair, skin, nails, or genital areas of a mammal.

For example, the topical agent may be selected from sunscreens, moisturizers, anti-microbial agents, anti-fungals, anti-inflammatory agents, anti-mycotic agents, anti-parasite agents, skin lightening agents, skin pigmentation darkening agents, anti-acne agents, sebum modulators, shine control agents, external analgesics, non-UV absorbing photoprotectors, antioxidants, keratolytic agents, vitamins, nutrients, energy enhancers, i.e., carnitine, anti-perspiration agents, astringents, deodorants, hair removers, firming agents, anti-callous agents, and agents for hair, nail, or skin conditioning, as well as other ingredients that may be topically applied and combinations of the foregoing.

In one embodiment, the topical agent is selected from, but not limited to, the group consisting of hydroxy acids, benzoyl peroxide, D-panthenol, carotenoids, free radical scavengers, spin traps, retinoids such as retinol, retinaldehyde, and retinyl palmitate, ceramides, polyunsaturated fatty acids, essential fatty acids, enzymes, enzyme inhibitors, minerals, hormones such as estrogens, steroids such as hydrocortisone, 2-dimethylaminoethanol, copper salts such as copper chloride, peptides containing copper such as Cu:Gly-His-Lys, coenzyme Q10, amino acids such a proline, vitamins, lactobionic acid, acetyl-coenzyme A, niacin, riboflavin, thiamin, ribose, electron transporters such as NADH and FADH2, and other botanical extracts such as aloe vera, and derivatives, soya extracts, and mixtures thereof.

Such topical agents are typically present in the composition in an amount of from about 0.001 % to about 20% by weight of the composition, e.g., about 0.005% to about 10%, or about 0.01 % to about 5% by weight of the composition.

Examples of vitamins include, but are not limited to, vitamin A, vitamin Bs such as vitamin B3, vitamin B5, and vitamin B12, vitamin C, vitamin K, and vitamin E and derivatives thereof

Examples of hydroxy acids include, but are not limited, to glycolic acid, lactic acid, malic acid, salicylic acid, citric acid, and tartaric acid. See, e.g., European Patent Application No. 273,202.

Examples of antioxidants include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palpitate and ascorbyl polypeptide). Suitable oil-soluble antioxidants include, but are not limited to, butylated hydroxytoluene, retinoids (e.g., retinal, retinaldehyde, and retinyl palmitate), tocopherols (e.g., tocopherol acetate), tocotrienols, and ubiquinone. Suitable natural extracts containing antioxidants include, but are not limited to, extracts containing flavonoids and isoflavonoids and their derivatives (e.g., genistein and diadzein), extracts containing resveratrol and the like. Examples of such natural extracts include grape seed, green tea, pine bark, and propolis.

Various other agents may also be present in the composition, as long as they are compatible with the other ingredients in the composition. Such other agents may include, for example, humectants, proteins and polypeptides, chelating agents (e.g., EDTA), preservatives (e.g., parabens), and pH adjusting agents. In addition, the composition may contain conventional cosmetic adjuvants such as fragrances. Dyes (non-fluorescent), opacifiers, and pigments, may also be included in the composition as long as they do not interfere with the ability of the device to detect the topical agent in the composition.

The composition comprises a sunscreen and a fluorescent chromophore. Such composition preferably has an SPF of at least about 2, in particular about 2 to about 60, more particularly about 10 to about 60. The sunscreen may be present in an amount corresponding to about 2 to about 40 % by weight of the composition.

Sunscreens useful in the present invention are compounds that absorb, reflect, or scatter radiation in the UV range. These include W-A absorbers, W-B absorbers, inorganic pigment filters and infrared protectors. Sunscreens can be oil or water-soluble, that is, having a relative preference to solubilize in hydrophobic or hydrophilic materials.

Oil soluble UV-B absorbers include:
- 3-Benzylidene campher, specifically 3-benzylidene norcampher and derivatives thereof, e.g. 3-(4-methylbenzylidene) campher;
- 4-Aminobenzoic acid derivatives, specifically 4-(dimethylamino)benzoic acid-2-ethylhexyl esters, 4-(dimethylamino)benzoic acid-2-octyl esters and 4-(dimethylamino)benzoic acid amylesters;
- Esters of cinnamonic acid, in particular 4-methoxycinnamonic acid-2-ethylhexylester, 4-methoxycinnamonicacid propylester, 4-methoxycinnamonic acid isoamyl ester, 2-cyano-3,3-phenylcinnamonic acid-2-ethylhexyl ester (octocrylene);
- Esters of salicylic acid, i.e., salicylic acid-2-ethylhexylester, salicylic acid-4-isopropylbenzyl ester, salicylic acid homomenthyl ester;
- Derivatives ofbenzophenones, in particular 2-hydroxy-4-methoxybenzophenone, 2-hydroxy-4-methoxy-4'-methylbenzophenone, 2,2'-dihydroxy-4-methoxybenzophenone;
- Esters ofbenzalmalonic acid, in particular 4-methoxybenzmalonic acid di-2-ethylhexyl ester;
- Triazine derivatives, for example 2,4,6-trianilino-(p-carbo-2'-ethyl-1'-hexyloxy)-1,3,5-triazine and octyltriazone; or benzoic acid, 4,4'-[[6-[[[(1,1-dimethylethyl)amino]carbonyl]phenyl]amino]-1,3,5-triazine-2,4-diyl]diimino]bis-, bis(2-ethylhexyl) ester (UVASORB HEB);
- Propane-1,3-diones, for example, 1-(4-tert.butylphenyl)-3-(4'-methoxyphenyl)propane-1,3-dione;
- Ketotricyclo(5.2.1.0)decane derivatives.

Water-soluble UV-A and UV-B absorbers include for example:
- 2-Phenylbenzimidazol-5-sulfonic acid and its alkali-, alkaline earth-, ammonium-, alkylammonium-, alkanolammonium- and glucammonium salts;
- Sulfonic acid derivatives of benzophenones, in particular 2-hydroxy-4-methoxybenzophenone-5-sulfonic acid and its salts;
- Sulfonic acid derivatives of 3-benzylidene campher, e.g. 4-(2-oxo-3-bornylidene methyl)benzolsulfonic acid and 2-methyl-5-(2-oxo-3-bornylidene)sulfonic acid and its salts.

Typical UV-A absorbers include derivatives of benzoylmethane, for example, 1-(4'-tert.butylphenyl)-3-(4'-methoxyphenyl)propane-1,3-dione, 4-tert.-butyl-4'-methoxydibenzoylmethane (PARSOL 1789), 1-phenyl-3-(4'-isopropylphenyl)-propane-1,3-dione, derivatives of benzoic acid 2-(4-diethylamino-2-hydroxybenzoyl)-benzoic acid hexylester (UVINUL A+), or 1H-benzimidazole-4,6-disulfonic acid, 2,2'-(1,4-phenylene)bis-, disodium salt (NEO HELOPAN AP ).

Mixtures of UV-A and UV-B absorbers can also be used.

Of particular interest are the so-called broadband filters. One type of such filters are the water-soluble filters, more specifically the benzotriazoles, in particular the benzotriazole derivative known as 2,2'-methylene-bis-(6-(2H-benzotriazole-2-yl)-4-(1,1,3,3-tetramethylbutyl)-phenol) [INCI: Bisoctyltriazol], which is commercially available under the tradename TINOSORB M from CIBA Chemicals. Another useful benzotriazole derivative is 2-(2H-benzotriazole-2-yl)-4-methyl-6-[2-methyl-3-[1,3,3,3-tetramethyl-1-[(trimethylsilyl)oxy]disiloxanyl]propyl]-phenol (CAS-No.: 155633-54-8) also identified by the INCI name drometrizole trisiloxane and available from Chimex under the tradename MEXORYL XL. These benzotriazole derivatives can be conveniently incorporated in the water phase at a pH above 4.5.

Other useful water-soluble UV absorbers are the sulfonated UV filters such as 3,3'-(1,4-phenylenedimethylene)bis(7,7-dimethyl-2-oxo-bicyclo-[2.2.1]hept-1-yl methanesulfonic acid, and its sodium, potassium, or its triethanolammonium salts, and the sulfonic acid itself, identified by the INCI name terephthalidene dicamphor sulfonic acid (CAS No. 90457- 82-2), which is available, for example, under the trade name MEXORYL SX from Chimex.

Oil-soluble broadband filters include the asymmetrically substituted triazine derivatives. Of particular interest is 2,4-bis-{[4-(2-ethyl-hexyloxy)-2-hydroxy]-phenyl}-6-(4-methoxyphenyl)-1,3,5-triazine (INCI: anisotriazine) that is commercially available under the tradename TINOSORB S from CIBA Chemicals.

Examples of inorganic pigment filters include insoluble pigments, namely finely dispersed metal oxides or metal salts. Examples of useful metal oxides in particular are zinc oxide and titanium dioxide as well as oxides of iron, zirconium, silicon, manganese, aluminium and cerium as well as mixtures thereof. Salts that can be used comprise silicates (talcum), barium sulfate, or zinc stearate. The particle size of these pigments is sufficiently small, e.g. less than 100 nm, in particular between 5 and 50 nm and more in particular between 15 and 30 nm. The particles may be spherical or may have other shapes, such as ellipsoidal or another similar shape. The surface of the pigments may have been treated, e.g. hydrophilized or made hydrophobic. Typical examples are coated titanium dioxide, e.g. titanium dioxide T 805 (available from Degussa) or EUSOLEX T 2000 (Merck). Silicones can be used as hydrophobic coating agents, in particular trialkoxyoctyl silanes or simethicones. So-called micro- or nanopigments are particularly attractive for use as sunscreens.

The composition also comprises a fluorescent chromophore. "Fluorescent chromophore" means a compound that absorbs radiation (e.g., light) at one wavelength (its wavelength of excitation) and re-emits radiation at a higher wavelength (its wavelength of emission). The wavelength of excitation is generally a wavelength at which the absorption has a peak value. The wavelength of emission is separated from (i.e., greater than) the wavelength of excitation by an amount (in nanometers) known as the "Stoke's shift."

In one embodiment of the invention, when measured at the wavelength of excitation of the fluorescent chromophore, the absorbance of the fluorescent chromophore is at least 5 times greater than the absorbance of the topical agent(s) in the composition alone or in combination.

As will be recognized by one skilled in the art, "absorbance" means the logarithm of the ratio of intensity of incident light prior to being transmitted through a medium to the intensity of light that is transmitted through the medium. The absorbance is a function of the medium as well as the path length through which the light travels and the concentration of the absorbing entity within the medium. In order to calculate absorbance of the fluorescent chromophore in a particular test composition, a film having a thickness from about 5 microns to about 10 microns is cast from the test composition (to get a "baseline" absorbance) as well as from a composition that is identical to the test composition, except that the fluorescent chromophore is removed. The films may be cast on a suitable substrate (e.g., PMMA) that is substantially transparent at the wavelengths of interest. A UV-VIS spectrophotometer, such one of those commercially available from Labsphere, is suitable to measure absorbance from cast firms. By inputting the parameters on the software that controls the spectrophotometer, any variation in film thickness is accounted for and normalized.

Similarly, in order to calculate an absorbance of the topical agent(s) in a test composition, a film having a thickness from about 5 microns to about 10 microns is cast from a composition that is identical to the test composition, except that the topical agents are removed. If the one or more topical agents comprise more than 5% of the test composition, then, in order to maintain the a constant ratio of other ingredients in the composition, a diluent that is transparent to the wavelengths in question should be added to the composition to compensate for the missing topical agents. Absorbance is calculated using the same equipment. The ratio of absorbance of the fluorescent chromophore to absorbance of the topical agent is then calculated by division.

The fluorescent chromophore has a water solubility that is similar to the topical agent(s). If the fluorescent chromophore and topical agent have similar water solubilities, the fluorescent chromophore and the topical agent will tend to be removed at a similar rate from a surface such as the skin when exposed to water and moisture. Accordingly, the fluorescent chromophore may be used as a "proxy" or "marker" for the topical agent. Detection of absorbance of the fluorescent chromophore correlates well with the concentration or presence of the topical agent in the composition.

Accordingly, in a further embodiment, in order to provide compatibility or association of the fluorescent chromophore with topical agents that are hydrophobic or have low water solubility, the fluorescent chromophore has a water solubility that is less than about 2% by weight, such as less than about 1 % by weight.

The fluorescent chromophore is a naphthalene derivative. The polyaromatic compound may be substituted with one or more functional groups that confer at most, only slight water solubility (e.g., cyclic or aliphatic groups such as imine, amine, alkyl groups, esters, ethers, and, combinations thereof). One example of a suitable class of compounds is naphthalimides, e.g., naphthalene that has been substituted with a cyclic imide.

One notable naphthalimide is n-butyl-4-(butylamino)1,8-naphthalimide, also known as FLUROL 555, commercially available as DFSB-K43 from Risk Reactor of Huntington Beach, California. This compound has a wavelength of excitation of about 450 nm and a wavelength of emission of about 500 nm.

In a further embodiment of the invention, the fluorescent chromophore has a wavelength of excitation that is less than about 500 nm. Such fluorescent chromophores are particularly useful in that they may be used in conjunction with detection systems employing a detection light source having a wavelength less than 500 nm. Such lower wavelengths may be suitable for detection in an environment of bight sunlight for example. Higher wavelengths are less efficiently absorbed by the skin, and are more likely to undergo interference with external sunlight that is incompletely shrouded.

The fluorescent chromophore has a wavelength of excitation that falls within a particular range. This is desirable because typical sunscreens will absorb strongly in the ultraviolet and often absorb or scatter strongly in the lower wavelengths of the visible spectrum. Futhermore, because interference with ambient light is more pronounced at higher wavelengths, the wavelength of excitation of the fluorescent chromophore is preferably not too high.

The topical agent comprises a sunscreen and the wavelength of excitation of the fluorescent chromophore is greater than about 400 nm. The wavelength of excitation of the fluorescent chromophore may be in the visible spectrum, such as between about 400 nm and about 600 nm, more preferably between about 400 nm and about 500 nm, most preferably between about 450 nm and about 500 nm.

In one embodiment, the fluorescent chromophore has a water solubility that is less than about 2% by weight, such as less than about 1% by weight.

The compositions of the present invention are suitable for topical application to a variety of surfaces, including hair, skin, nails, and genital areas. In one embodiment, the composition is spreadable across the skin in order to deliver the topical agent and fluorescent chromophore thereto. Furthermore, for aesthetic purposes, in one embodiment, the amount of fluorescent chromophore in the composition is sufficiently low such that the composition is capable of being topically applied to skin in a generous manner, without imparting color (such as by "staining" the skin, which may be unsightly) to the skin after it has been completely rubbed in.

In one embodiment, the composition comprises about 0.001 to about 0.1 weight percent fluorescent chromophore.

The compositions may be made into a wide variety of product types that include but are not limited to lotions, creams, gels, sticks, sprays, ointments, shampoos, pastes, mousses, and cosmetics. These product types may comprise cosmetically acceptable carrier systems including, but not limited to solutions, emulsions, gels, solids and liposomes.

Compositions of the invention formulated as solutions typically include an aqueous (e.g., water) or organic solvent (e.g., from about 80% to about 99.99% or from about 90% to about 99% of an acceptable aqueous or organic solvent). Examples of suitable organic solvents include: propylene glycol, polyethylene glycol, polypropylene glycol, glycerol, 1,2,4-butanetriol, sorbitol esters, 1,2,6-hexanetriol, ethanol, butylenes glycol, and mixtures thereof.

Compositions of the invention formulated as solutions may comprise one or more emollients. Such compositions typically contain from about 2% to about 50% of an emollient(s). As used herein, "emollients" refer to materials used for the prevention or relief of dryness, as well as for the protection of the skin.

Lotions typically comprise from about 1% to about 20% (e.g., from about 5% to about 10%) of an emollient(s) and from about 50% to about 90% (e.g., from about 60% to about 80%) of water.

The composition may also be formulated as a cream. A cream typically comprises from about 5% to about 50% (e.g., from about 10% to about 20%) of an emollient(s) and from about 45% to about 85% (e.g., from about 50% to about 75%) of water.

The composition may also be formulated as an ointment. The ointment may comprise a simple base of animal or vegetable oils or semi-solid hydrocarbons (oleaginous, absorbent, emulsion and water soluble ointment bases). Ointments may also comprise absorption ointment bases that absorb water to form emulsions. Ointment carriers may also be water-soluble. An ointment may comprise from about 2% to about 10% of an emollient(s) plus from about 0.1% to about 2% of a thickening agent(s).

If the carrier system is formulated as an emulsion, typically from about 1% to about 10% (e.g., from about 2% to about 5%) of the carrier system comprises an emulsifier(s). Emulsifiers may be nonionic, anionic or cationic.

Lotions and creams can be formulated as emulsions. Typically such lotions comprise from 0.5% to about 5% of an emulsifier(s). Such creams would typically comprise from about 1% to about 20% (e.g., from about 5% to about 10%) of an emollient(s); from about 20% to about 80% (e.g., from 30% to about 70%) of water; and from about 1% to about 10% (e.g., from about 2% to about 5%) of an emulsifier(s).

Single emulsion skin care preparations, such as lotions and creams, of the oil-in-water type and water-in-oil type, are well-known in the cosmetic art and are useful in the subject invention. Multiphase emulsion compositions, such as the water-in-oil-in-water type, are also useful in the subject invention. In general, such single or multiphase emulsions contain water, emollients, and emulsifiers as essential ingredients.

In one embodiment, the composition may be in the form of oil in water (O/W) emulsion. O/W emulsions contain an oil phase that may comprise suitable oils that are skin-compatible components or mixtures that are non-water miscible. Preferably, the oils are liquid at ambient temperature, in particular are liquid at 25° C. They can contain certain amounts of solid lipid components (e.g. fats or waxes) as long as the complete oily mixture is liquid at ambient temperature or at the temperature mentioned above.

The water phase in the O/W emulsions may be pure water but usually contains one or more hydrophilic components. The latter can be lower alkanols, polyols, water-soluble active ingredients, preservatives and moisturizers, chelating agents, etc.

Applicants have noted that it is desirable for the fluorescent chromophore and the topical agent whose presence or concentration is desirable to be detected to co-exist in the same phase of the composition. As such, it is desirable to prepare the composition by mixing the topical agent and the fluorescent chromophore such that the topical agent and the fluorescent chromophore are homogeneously co-solublized. a method of making compositions of the present invention includes mixing a fluorescent chromophore, a sunscreen, and an optional diluent, sufficiently to form a single phase composition. "Single phase" composition means a composition in which the sunscreen and the fluorescent chromophore are substantially homogeneous on a molecular level.

The diluent is generally a compound that is capable of dissolving both the sunscreen and the fluorescent chromophore. Since the sunscreen and the fluorescent chromophore have low water solubility, the diluent is a hydrophobic material, such as, for example, mineral oils, petrolatum, vegetable oils (glyceryl esters of fatty acids, triglycerides), waxes and other mixtures of esters, not necessarily esters of glycerol; polyethylene and non-hydrocarbon based oils such as dimethicone, silicone oils, silicone gums, and the like. Alternatively, the diluent may have mixed hydrophobic and hydrophilic character, for instance a solvent such as an alcohol like isopropanol.

Additional components may be added to the single phase composition, i.e., added into a vessel containing the single phase composition, or vice versa. The mixing of the single phase composition and the additional components may result in a composition having multiple phases, i.e., a stable multi-phase composition, such as an emulsion as described above, a dispersion, an aerosol, etc.

In another embodiment, the additional components are free of sunscreens and fluorescent chromophores, although this is not required.

The compositions may be optionally prepared using a mineral water, for example mineral water that has been naturally mineralized such as EVIAN Mineral Water (Evian, France). In one embodiment, the mineral water has a mineralization of at least about 200 mg/L (e.g., from about 300 mg/L to about 1000 mg/L). In one embodiment, the mineral water contains at least about 10 mg/L of calcium and/or at least about 5 mg/L of magnesium.

The composition may be topically applied by means of spreading on skin, nails, hair, or genital areas, e.g., by use of the hands, or an applicator such as a wipe, roller, or spray. Depending on the selection of the topical agent, the compositions can be employed for a number of end uses, such as photoprotection, moisturization, cleansing, acne, mottled hyperpigmentation, age spots, wrinkles, fine lines, cellulite, and other visible signs of aging (whether due to photoaging or chronoaging).

According to the invention, the presence of the composition on a surface may be determined by directing light onto the composition to excite the fluorescent chromophore contained in the composition. The light directed onto the composition should have a wavelength corresponding to the wavelength of excitation of the fluorescent chromophore. The light emitted from the excited fluorescent chromophore, which has a longer wavelength, may then be collected, and the level of fluorescence determined. Optionally, this level can be compared with a predetermined level to evaluate not only whether the composition is present on the surface, but whether a sufficient amount is present.

Referring to the Figure, in one embodiment, determination of the presence and/or amount of a composition 6 comprising a fluorescent chromophore 7 on a surface is carried out using a device according to the invention. The device comprises a light emitter 2, a light detector 3, an electronic evaluation system 4, and a display system 5. These elements are linked electronically, and may be contained in a single housing 1. In a further embodiment, such housing is hand-held and battery powered for ease of use by a consumer. All of the elements of the device may be obtained from commercial sources, and will be familiar to those skilled in the art of consumer and cosmetic devices.

The light emitter 2 comprises a means for directing light onto a surface. In one embodiment, the light emitter directs visible light onto the surface. This is advantaegous, for example, for use with a composition comprising a sunscreen, in that the ultraviolet filters of the sunscreen will not interfere with operation of the device.

The light emitter 2 provides light and may be a pulsed or continuous wave source that is generally narrowband (spectrally concentrated), such as a light emitting diode (LED) or laser. The LED or laser is constructed from materials known in the art (e.g., compound semiconductor materials) such that it emits in a particular wavelength or range of range of wavelengths that encompasses the wavelength of excitation of the fluorescent chromophore. Intensity of the excitation energy may be in the range of 1 mW or less. The emitted light may be subsequently filtered, attenuated, amplified, polarized, or otherwise modified by one or more optical elements before it reaches an expanse of skin to which it is directed. At the point which the light reaches an outer surface of the expanse of skin, it interacts with the skin and any with composition that has been applied to thereto.

Fluorescent chromophore present on the skin is optically excited by the emitted light, resulting in fluorescence to be emitted. The fluorescent light enters the opening of the device and is optically directed (via mirrors, lenses, or light conductive media) towards the light detector such that the presence or amount of fluorescent chromophore on the skin (and, indirectly, the presence or amount of topical agent) may be determined. For instance, in the Figure, before reaching the detector, the fluorescent light is redirected by a mirror 9 to pass through a blocking filter 8, designed to permit passage of essentially only wavelengths close the wavelength of emission of the fluorescent chromophore. The blocking filter generally comprises one or more materials that are transparent to wavelengths at or near the wavelength of emission of the fluorescent chromophore, but are highly absorbing for other wavelengths. Suitable blocking filters are commercially available from such vendors as Oriel Optics of Stratford, Connecticut, among other vendors.

The light detector 3 may for example be a photodetector, such as one known in the art for detecting light signals. The light detector is tuned to absorb at or near the wavelength of emission of the fluorescent chromophore.

The electronic evaluation system 4 is linked to the light detector, and comprises means for calculating an algorithm. The algorithm may simply relate the receipt of light by the light detector to the presence of composition on a surface, or it may additionally calculate the amount of composition on a surface using the amount of light received by the light detector. Alternatively, the algorithm may be used to compare the amount of light received by the light detector with a predetermined amount, and thereby calculate whether a sufficient, minimum level of composition is present on the surface.

Output from the electronic evaluation system is sent to the display system 5, which provides a visual display of the output from the electronic system. The display system may employ a digital or analogue format. It may comprise a simple LED indicator, for example, wherein green indicates the presence of composition or an adequate amount of composition, and red indicates the absence of composition or the presence of an inadequate amount of composition. Alternatively, the display system may display other colors, numbers, letters, or other indicia indicating the actual or relative amount of composition on the surface.

In one embodiment, the device further comprises a shroud 10 proximal the light emitter. The shroud is capable of shielding the area of the surface being exposed to light from the light emitter. The shielding ability of the shroud may arise from, for example, the shroud's ability to absorb light well throughout the visible (and optionally, near UV) spectrum. In this manner, ambient light cannot interfere with operation of the device. The shroud may be of varying geometries such as, for example, a cylinder of black or dark-colored material that extends from the device. While the shroud may be of varying construction, in one embodiment of the invention, the shroud includes a skin-contactable portion that is formed from a resilient material, such as an elastomer that is capable of deforming when lightly pressed against the skin such that it conforms to the curves of the skin around the area to be scanned. As such a gasket or barrier is created that prevents ambient light from reaching the photodetector. The elastomeric material is preferably constructed such that walls of the cylinder do not readily collapse or buckle under the stress of pressing the elastomeric material against the skin (causing the undesirable effect of allowing ambient light in during scanning of the skin).

The device may be configured for use with a single type of composition. Alternatively and advantageously, the device may be configured for use with multiple compositions. That is, the device may be set to emit and receive light at certain, preset wavelengths. Different compositions formulated with different levels of fluorescent chromophore may be employed with the device. For example, the device may be compatible with a) a first composition that includes a topical sunscreen agent and a first concentration of fluorescent chromophore; and b) a second composition comprising the topical agent a second concentration of the fluorescent chromophore, wherein the first concentration is substantially greater than the first concentration.

For example, the first composition may be a daily wear SPF product such as a moisturizer plus a sunscreen. Such a product may be designed for a first sunlight exposure environment that is relatively low intensity. The second composition may be a recreational suncare product (designed for use in a second sunlight exposure environment that is more intense than the first sunlight exposure environment and where suncreens are typically prone to greater loss of adhesion to the skin from exposure to water, sweating, or rubbing from sand or towels). It is therefore more critical that sunscreen adhesion to the skin be more closely monitored with the device in the latter case, where there is more intense sunlight exposure and a higher likelihood of significant skin damage if insufficient sunscreen is on the skin. As such, a single device that is designed to detect the presence of a fixed, minimum level of fluorescent chromophore may be used with either the first composition or the second composition.

Kits comprising the compositions of the invention and optionally the device described above are now described: In one embodiment, the kit comprises: a) a composition comprising a topically active agent and a fluorescent chromophore; and b) a device for determining the presence of the composition on a surface, which device comprises a light emitter, a light detector, an electronic evaluation system to determine the level of fluorescence of the fluorescent chromophore, and a display system. The topically active agent is a sunscreen.

The kit may comprise one or more than one composition or the same or different type. The composition(s) may be put into finished packaged form such as inside containers made of paper, plastic, metal, or glass, i.e., tubes or jars. The kit may comprise additional packaging such as a plastic or cardboard box for storing the container(s) and the device. The kit may further contain instructions for using the composition(s) and the device. Such instructions may be printed on a container, label insert, or on any additional packaging.

### Example 1

A composition according to the invention was made by adding 0.1 g of DFSB-K43 from Risk Reactor of Huntington Beach, California to 100 g of SUNDOWN SPF 60 sunscreen product. The composition was then applied to the surface of a ground surface PMMA plate at densities ranging from 0.5, 1.0, 1.5, 2.0, and 2.5 mg/cm² (approximately 1 "square test sites). The fluorescence of the DFSB-K43 in the applied sunscreen was measured by exciting the of DFSB-K43 with 450nm radiation (from a monochromator), and measuring the emission fluorescence at 500nm. The fluorescence intensity over the test area was measured using 4 individual measurements, and the results are plotted below.

The fluorescence intensity was highly correlated with the application density of the composition r² = 0.927 indicating that this technique is indicative of product application density and is predictive of product application quantity.

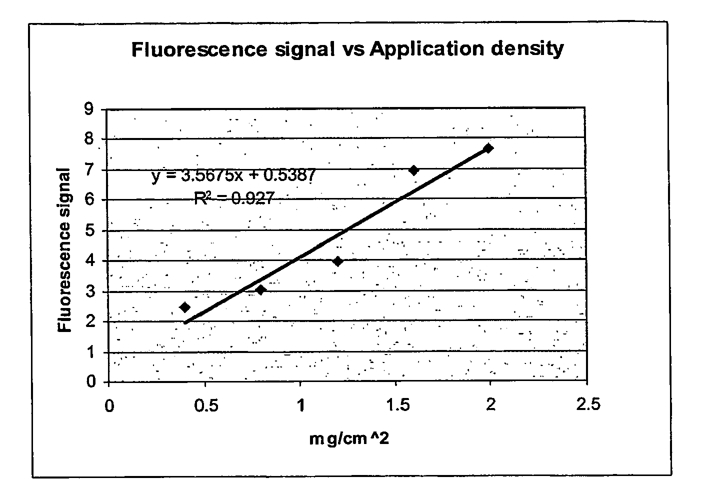

### Example 2 (reference example)

The same sample preparation was measured using conventional in vitro SPF measurement equipment, Labsphere UV spectrophotometer, to evaluate the SPF of each of the application density samples. The relationship between the fluorescence signal of the fluorescent chromophore in the sunscreen is shown to clearly correlate with the SPF of the product on the plate.

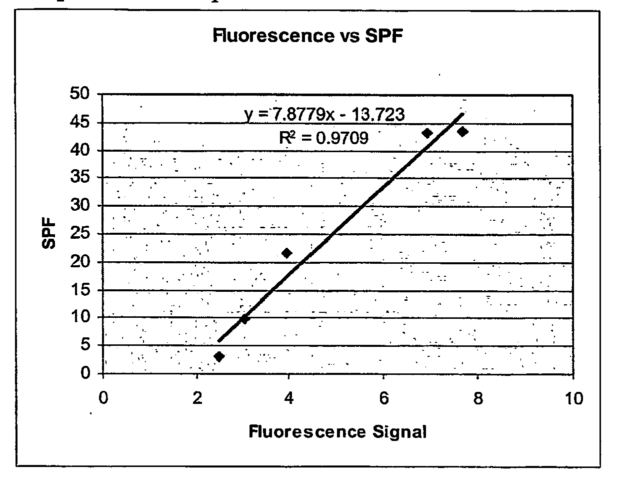

In this example, a threshold fluorescent level of at least 6 would be required to indicate that sufficient sunscreen had been applied to the skin. The diagnostic tool would then indicate a "Yes" signal, that sufficient sunscreen was in place. The indicator may be a green glowing light, or a LCD indicator, or a "meter" showing "Good". Fluorescence below this value of 6 would signal insufficient sunscreen coverage with a "No" signal such as a red light, a "no" LCD indicator or a meter showing "Not Enough" for example.

### Example 3 (reference example)

A sample of an SPF 30 sunscreens preparation containing the 0.1% Yellow Dye #43 was prepared on a ground surface PMMA plate at a density of 1.6 mg/cm2 and allowed to dry for approximately 10 minutes. The SPF of the sample and the fluorescence signal from the sample were measured as above with both the spectrofluorimeter device, and the Labsphere SPF spectrophotometer. After the initial measurements, the sample was placed under vigorously running tap water for ten to 15 seconds, and rubbed lightly with a fingertip in the stream of water, and the measurements were repeated again. The fluorescence signal decreased after washing/rubbing, indicating that some of the fluorescent chromophore was removed. However, it can be noted that the slope of the line in Example 2 is very similar to Example 1 (within about 12%), indicating that the fluorescence chromophore and UV-filter are removed in approximately the same proportion. This suggests that the fluorescence chromophore and UV-filter associate well with one another and that the device can predict the change in presence of the UV -filter from the change in fluorescence signal.

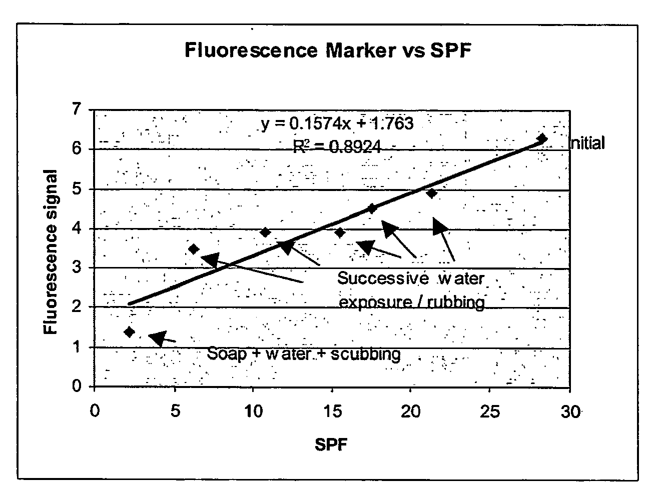

## Claims

1. A topical composition comprising one or more oil soluble ultraviolet sunscreen agents and a fluorescent chromophore, wherein the fluorescent chromophore is oil soluble, has a wavelength of excitation greater than 400nm, and is selected from a naphthalene derivative.

2. The composition according to claim 1, wherein the fluorescent chromophore is a naphthalimide.

3. The composition according to claim 2, wherein the fluorescent chromophore is n-butyl-4-(hutylamino) 1,8-naphthalimide having a wavelength of excitation of about 450nm.

4. The composition according to any preceding claim, wherein the sunscreen agent is present in the composition in an amount of from 2% to 40% by wight of the composition.

5. The composition according to any preceding claim, wherein the fluorescent chromophore is present in an amount of from 0.001 to 0.1 weight percent.

6. The composition according to any preceding claim, wherein the sunscreen agent and the fluorescent chromophore co-exist in the same phase of the composition.

7. The composition according to claim 6, wherein the composition is a single phase composition.

## Patentansprüche

1. Topische Zusammensetzung, die ein oder mehrere öllösliche Ultraviolett-Sonnenschutzmittel und ein fluoreszierendes Chromophor umfasst, wobei das fluoreszierende Chromophor öllöslich ist, eine Anregungswellenlänge von mehr als 400 nm besitzt und ausgewählt ist aus einem Naphthalin-Derivat.

2. Zusammensetzung nach Anspruch 1, wobei das fluoreszierende Chromophor ein Naphthalimid ist.

3. Zusammensetzung nach Anspruch 2, wobei das fluoreszierende Chromophor n-Butyl-4-(butylamino)-1,8-naphthalimid mit einer Anregungswellenlänge von etwa 450 nm ist.

4. Zusammensetzung nach einem vorangehenden Anspruch, wobei das Sonnenschutzmittel in der Zusammensetzung in einer Menge von 2 bis 40 Gew.-% der Zusammensetzung vorliegt.

5. Zusammensetzung nach einem vorangehenden Anspruch, wobei das fluoreszierende Chromophor in einer Menge von 0,001 bis 0,1 Gewichtsprozent vorliegt.

6. Zusammensetzung nach einem vorangehenden Anspruch, wobei das Sonnenschutzmittel und das fluoreszierende Chromophor gemeinsam in derselben Phase der Zusammensetzung vorkommen.

7. Zusammensetzung nach Anspruch 6, wobei die Zusammensetzung eine einphasige Zusammensetzung ist.

## Revendications

1. Composition topique comprenant un ou plusieurs agents antisolaires filtrant les rayons ultraviolets solubles dans l'huile et un chromophore fluorescent, dans laquelle le chromophore fluorescent est soluble dans l'huile, a une longueur d'onde d'excitation supérieure à 400 nm, et est choisi parmi les dérivés du naphtalène.

2. Composition selon la revendication 1, dans laquelle le chromophore fluorescent est un naphtalimide.

3. Composition selon la revendication 2, dans laquelle le chromophore fluorescent est un n-butyl-4-(butylamino)-1,8-naphtalimide ayant une longueur d'onde d'excitation d'environ 450 nm.

4. Composition selon l'une quelconque des revendications précédentes, dans laquelle l'agent antisolaire est présent dans la composition en une quantité de 2 à 40 % en poids de la composition.

5. Composition selon l'une quelconque des revendications précédentes, dans laquelle le chromophore fluorescent est présent en une quantité de 0,001 à 0,1 % en poids.

6. Composition selon l'une quelconque des revendications précédentes, dans laquelle l'agent antisolaire et le chromophore fluorescent coexistent dans la même phase de la composition.

7. Composition selon la revendication 6, dans laquelle la composition est une composition monophasique.
